# EUROPEAN PATENT APPLICATION

(11) **EP 3 573 072 A1**
(43) Date of publication of application: **27.11.2019**
(21) Application number: 18173477.3
(22) Date of filing: 22.05.2018
(51) Int. Cl.: G16H 50/30, G16H 50/20

(54) **PERFORMING A PROGNOSTIC EVALUATION**

(71) Applicant: Koninklijke Philips N.V., 5656 AE Eindhoven (NL)
(72) Inventor: VOS, Pieter Christiaan, 5656 AE Eindhoven (NL); HOFFMANN, Ralf, 5656 AE Eindhoven (NL); HENDRIKS, Monique, 5656 AE Eindhoven (NL)
(74) Representative: de Haan, Poul Erik

(57) **Abstract**

The invention discloses apparatus for performing a prognostic evaluation of a subject potentially having prostate cancer. The apparatus comprises a memory comprising instruction data representing a set of instructions; and a processor configured to communicate with the memory and to execute the set of instructions, wherein the set of instructions, when executed by the processor, cause the processor to obtain a subject profile (102) associated with the subject; obtain clinical data (104) associated with the subject; obtain imaging data (106) acquired in respect of the subject's prostate; obtain pathological information (108) relating to a biopsy acquired in respect of the subject's prostate; determine, based on at least the subject profile, the clinical data, the imaging data and the pathological information, a prognostic score (110, 112) relating to the cancer. Computer-implemented methods and a computer program product are also disclosed.

## Description

### FIELD OF THE INVENTION

The invention relates to an apparatus and method for performing a prognostic evaluation of a subject, particularly a subject having prostate cancer.

### BACKGROUND OF THE INVENTION

Prostate cancer is the most common cancer in men. Screening of prostate cancer has led to a reduction in advanced disease and disease-specific mortality, but has also led to over-diagnosis of cases that would not have caused death if left untreated.

Cancer is described in terms of a "stage" which refers to the extent of the cancer, such as a size of a cancerous tumor, and the extent to which the cancer has spread. Correct staging of prostate cancer can help to prevent over-diagnosis, as the stage assigned when the cancer is diagnosed may be used in determining which treatment to administer. To characterize prostate cancer, a group of specialists are provided with details of the patient and details of the cancer. The specialists are required to correctly interpret the cancer status and predict a possible outcome (e.g. a likelihood that the patient die from the disease in his lifetime). Combining a large amount of available data from different sources in a cross-domain setting is a difficult and subjective task and is therefore prone to errors. For example, the analysis of one specialist may differ from the analysis of another specialist reviewing the same case. This may, for example, lead to conflicting views of how the patient should be treated.

A scoring mechanism referred to as Prostate Imaging Reporting and Data System (PI-RADS) has been developed to reduce observer variability in reporting cases of prostate cancer, and to improve the cancer staging performance. The PI-RADS scoring system provides a structured way of interpreting multi-parametric magnetic resonance imaging (MRI) data. While PI-RADS provides good prognostic value, a PI-RADS score might not be perfect as variability between observers (e.g. specialists allocating a PI-RADS score to a particular lesion) may exist due to differences in experience. Based on a PI-RADS score alone, the positive predictive value (PPV) (i.e. the proportion of cases that are considered to have cancer based on analysis of an MRI scan which are found to be true-positives) is relatively low. In other words, using just the PI-RADS scoring system, many patients may be wrongly diagnosed as having life-threatening prostate cancer.

There is, therefore, a need for an improved mechanism for providing a prognostic evaluation of a subject suffering from prostate cancer. Moreover, there is a need for a prognostic evaluation mechanism which avoids, or at least reduces the number of occurrences of, false diagnoses.

### SUMMARY OF THE INVENTION

Using the present invention, a more accurate prognostic evaluation of a subject having prostate cancer can be made when various factors are taken into account. Various factors, or features, have been identified which, when combined, can be used to calculate a score indicative of the severity and/or the aggressiveness of the cancer. The determined score can be used to aid the decision regarding which, if any, treatment should be administered in respect of the cancer.

According to a first aspect, the invention provides an apparatus for performing a prognostic evaluation of a subject having prostate cancer, the apparatus comprising a memory comprising instruction data representing a set of instructions; and a processor configured to communicate with the memory and to execute the set of instructions, wherein the set of instructions, when executed by the processor, cause the processor to obtain a subject profile associated with the subject; obtain clinical data associated with the subject; obtain imaging data acquired in respect of the subject's prostate; obtain pathological information relating to a biopsy acquired in respect of the subject's prostate; determine, based on at least the subject profile, the clinical data, the imaging data and the pathological information, a prognostic score relating to the cancer.

By functioning in this way, the processor is able to serve as a prognostic evaluation unit, capable of outputting an accurate score relating to the cancer, based on a particular set of input features. The outputs provided by such an apparatus may be used by a medical professional to determine an appropriate course of action. The apparatus provides the medical professional with a valuable insight into the cancer which may, for example, result in surveillance (i.e. non-surgical) action being taken rather than costly surgical action, when surgery is not justified.

In some embodiments, the determined prognostic score may comprise a score relating to the likelihood that the cancer is clinically significant. In other embodiments, the determined prognostic score may comprise a score relating to the likelihood that the cancer is non-localized. In some embodiments, the prognostic score may comprise both a score relating to the likelihood that the cancer is clinically significant and a score relating to the likelihood that the cancer is non-localized, or a combination of both scores.

The cancer may be considered to be non-localized if, following a radical prostate domain, a pathologic stage relating to any remaining cancer would be greater than pT2.

In some embodiments, the subject profile may comprise one or more of: an age of the subject; a family history of cancer; demographic data for the subject; an ethnic background of the subject; comorbidities of the subject; and a treatment history of the subject.

The clinical data may, in some embodiments, comprise one or more of: prostate-specific antigen (PSA) density data; clinical tumor stage information; data relating to an outcome of a digital rectal exam; and data relating to an outcome of a transrectal ultrasound.

In some embodiments, the imaging data may comprise one or more of: a Prostate Imaging Reporting and Data System (PI-RADS) score relating to the prostate cancer; spatial information relating to a lesion associated with the prostate cancer; and apparent diffusion coefficient (ADC) values relating to a lesion associated with prostate cancer. The PI-RADS score and the spatial information may be derived from multi-parametric magnetic resonance imaging information.

The imaging data may, in some embodiments, comprise a PI-RADS score relating to each of one or more lesions associated with the prostate cancer, each PI-RADS score determined using one or more of T2-weighted image data, diffusion-weighted image data and dynamic contrast enhanced image data. The spatial information may comprise an indication of a size of the one or more lesions in total, in an anterior region of the subject's prostate, in a posterior region of the subject's prostate; in a peripheral zone of the prostate, in a central zone of the prostate, in a transition zone of the prostate and/or in an anterior fibromuscular stroma of the prostate.

In some embodiments, the set of instructions, when executed by the processor, may cause the processor to determine the prognostic score by using a prediction model. For example, in some embodiments, the set of instructions, when executed by the processor, may cause the processor to determine the prognostic score by using a trained random forest classifier or a regression analysis model.

The set of instructions, when executed by the processor, may cause the processor to determine the prognostic score by using a trained random forest classifier. The random forest classifier may be arranged to classify input data based on a subset of a plurality of features. The subset of features may include those feature of the plurality of features which have the greatest impact in the classification.

According to a second aspect, the invention provides a computer-implemented method of performing a prognostic evaluation of a subject potentially having prostate cancer, the method comprising obtaining a subject profile associated with the subject; obtaining clinical data associated with the subject; obtaining imaging data acquired in respect of the prostate; obtaining pathological information relating to a biopsy acquired in respect of the subject's prostate; and determining, based on at least the subject profile, the clinical data, the imaging data and the pathological information, a prognostic score relating to the cancer.

In some embodiments, determining a prognostic score may comprise providing the subject profile, the clinical data, the imaging data and the pathological information as inputs to a prediction model; and obtaining as an output at least one of: a score indicating the likelihood that the cancer is clinically significant; and a score indicating the likelihood that the cancer is non-localized.

According to a third aspect, the invention provides a computer-implemented method of training a classifier to determine a prognostic score relating to prostate cancer in a subject based on a subject profile associated with the subject, clinical data associated with the subject, imaging data acquired in respect of the prostate and pathological information relating to a biopsy acquired in respect of the subject's prostate, the method comprising training the classifier using a set of training data; identifying a plurality of features which affect the output of the classifier; ranking the plurality of features according to their impact on the output of the classifier; disregarding the lowest-ranked feature of the plurality of features to obtain a subset of higher-impact features; and re-training the classifier on the basis of the subset of features.

In some embodiments, the method may further comprise down-sampling a majority class of the training data used to train the classifier such that the majority class is statistically balanced with a minority class of the training data. Training the classifier may comprise bootstrap sampling data in the minority class of the training data.

According to a fourth aspect, the invention provides a computer program product comprising a non-transitory computer-readable medium, the computer-readable medium having computer-readable code embodied therein, the computer-readable code being configured such that, on execution by a suitable computer or processor, the computer or processor is caused to perform the methods disclosed herein.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic illustration of an example of a mechanism or performing a prognostic evaluation of a subject having prostate cancer;
Fig. 2 is a simplified schematic of an example of an apparatus for performing a prognostic evaluation of a subject having prostate cancer, according to various embodiments;
Fig. 3 is a flowchart of an example of a method of performing a prognostic evaluation of a subject having prostate cancer, according to various embodiments;
Fig. 4 is a flowchart of an example of a method of training a classifier to determine a prognostic score, according to various embodiments;
Fig. 5 is a flowchart of a further example of a method of training a classifier to determine a prognostic score, according to various embodiments; and
Fig. 6 is a simplified schematic of an example of a machine-readable medium and a processor.

### DETAILED DESCRIPTION OF EMBODIMENTS

Embodiments disclosed herein relate to apparatuses and methods for performing a prognostic evaluation of a subject having prostate cancer. The prognostic evaluation involves determining a prognostic score relating to the cancer, which can be used, for example by a medical professional, to decide whether or not the cancer should be treated and, if so, the nature of the treatment to be administered. According to some embodiments disclosed herein, the prognostic score may be determined using a model or algorithm trained using machine learning techniques. Thus, one aspect of the invention relates to the training of an algorithm (e.g. a prediction model, such as a classifier) to provide a classifier trained to output a prognostic score based on various input features.

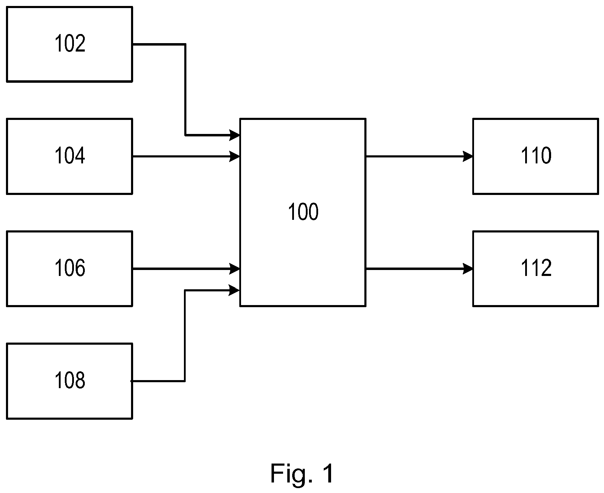

Fig. 1 shows, schematically, the flow of information according to various embodiments of the invention. Fig. 1 shows a prognostic evaluation unit 100 which may, for example, be implemented using computer software, or suitable a processing apparatus. For example, the prognostic evaluation unit 100 may be implemented using a computing device such as a desktop computer, a laptop computer, a tablet computer, a smart phone or a distributed computing system, for example in a cloud computing environment.

The prognostic evaluation unit 100 may be configured to receive data relating to the subject having prostate cancer, and the prognostic evaluation is based on this data. In the example shown in Fig. 1, the prognostic evaluation unit 100 is provided with patient characteristics 102 which may, for example, be in the form of a subject profile or patient profile; clinical data 104 associated with the subject, imaging data 106 relating to the prostate of the subject and pathological information 108 relating to a biopsy acquired in respect of the prostate of the subject.

The subject profile 102 associated with the subject having prostate cancer may include data acquired, for example, from an electronic health record associated with the subject. In some embodiments, the subject profile 102 may be generated manually, for example by a medical professional. The subject profile 102 may include characteristics of the patient or subject, such as the subject's age. The age of a subject suffering from prostate cancer may affect the likelihood of the prostate cancer being aggressive and/or spreading to other parts of the subject's body. In some examples, the subject profile 102 may include information indicative of a history of cancer in the subject's family. A subject having a family member who has suffered from prostate cancer previously may have a greater likelihood of suffering from an aggressive form of prostate cancer, for example. The subject profile 102 may, in some embodiments, include one or more of demographic data for the subject; an ethnic background of the subject; information relating to comorbidities of the subject; and a treatment history of the subject.

The clinical data 104 associated with a subject may include data relating to a cancerous lesion or tumor in, on or associated with the subject's prostate. In some examples, the clinical data 104 includes data indicative of a level of prostate-specific antigen (PSA), or a measure of PSA density, in the subject. The PSA density may be considered to be a ratio of the level of PSA (in ng/ml) measured in the blood relative to the prostate volume (in ml). While PSA is present in small quantities in men with healthy prostates, PSA levels increase when a subject's prostate suffers from a disease, such as prostate cancer. Thus, a relatively high level of PSA in a subject may be indicative that the subject is suffering from prostate cancer, and the level of PSA detected may be indicative of the severity of the cancer. The clinical data 104 may include the indication of the staging of the cancer as determined by a medical professional, for example when the cancer is initially diagnosed. For example, the clinical data 104 may include an indication of the clinical tumor stage, which is sometimes referred to as the clinical tumor class, or cT class. The clinical tumor stage is determined based on all of the information available to a medical professional before surgery is performed on a cancerous tumor. The clinical tumor stage is an indication of the extent to which the cancer has developed, for example through growth and spreading. An assessment of the clinical tumor stage is performed before any primary treatment of the cancer. The cT (or cTNM) staging notation is in accordance with the TNM Classification of Malignant Tumors system, as used by the American Joint Committee on Cancer (AJCC). In some embodiments, the clinical data may include one or more of data relating to an outcome of a digital rectal exam (DRE); and data relating to an outcome of a transrectal ultrasound (TRUS). A pathological tumor stage pT (or pTNM) is equivalent to the clinical tumor stage with the differences that the assessment is done after primary treatment of the cancer. The pT (or pTNM) staging notation is in accordance with the TNM Classification of Malignant Tumors system, as used by the American Joint Committee on Cancer (AJCC). Tumors may also be graded using a Gleason score. The Gleason score is based on how much the cancer looks like healthy tissue when viewed under a microscope by a pathologist, where less aggressive tumors generally look more like healthy tissue and tumors that are more aggressive are likely to grow and spread to other parts of the body.

The imaging data 106 relating to the subject's prostate may include imaging data acquired using known imaging modalities. In some examples, the imaging data 106 includes data acquired using a multi-parametric MRI modality. As noted above, multi-parametric MRI data may be used to designate a score to each lesion identified in an MR image, using the Prostate Imaging Reporting and Data System (PI-RADS) scoring format. A PI-RADS score is given based on the likelihood from the MR image that a lesion is malignant. Each identified lesion may be given PI-RADS score of between 1 and 5, where 1 indicates that the lesion is most probably benign and 5 indicates a high suspicion of malignancy.

Multi-parametric MRI techniques may provide various types of data by using different imaging techniques. A T2-weighted (T2-w) image highlights differences in the T2 (i.e. transverse) relaxation time of tissue being imaged. Diffusion-weighted imaging (DWI) allows visualization and analysis of the movement (diffusion) of water molecules in the intracellular space within the tissue being imaged. Dynamic contrast-enhanced (DCE) imaging allows visualization of vascularization (i.e. an increase of blood vessels) within the tissue being imaged. Imaging data (e.g. an image) may be acquired using each of these imaging techniques (i.e. T2-w, DWI and DCE), and a PI-RADS score may be given for a lesion visible in each image. In some embodiments, a PI-RADS score may be given for the dominant (e.g. the most prominent) lesion visible in each image. For T2-w images and DWI images, each lesion (or the most prominent lesion) is assigned a PI-RADS score on a scale of 1 to 5, as noted above. For DCE images in PI-RADS version 1 (v1), lesions are assigned a score on the scale of 1 to 5. For DCE images in PI-RADS version 2 (v2), lesions are scored with a yes (i.e. positive) or a no (i.e. negative). A "no" in the PI-RADS v2 system is translated to a 2 in the v1 system, and a "yes" in the PI-RADS v2 system is translated to a 4 in the v1 system. In this way, scores from PI-RADS v1 or v2 can be used. The scoring from the DCE images may be used to provide or modify a PI-RADS score provided based on the T2-w or DWI images.

PI-RADS scores assigned to lesions based on T2-w, DWI and DCE images are generally combined to give a single, overall PI-RADS score. However, according to embodiments disclosed herein, the individual PI-RADS scores may be used in order to perform the prognostic evaluation.

In some examples, the individual PI-RADS scores obtained using the T2-w, DWI and DCE techniques may be combined with spatial (e.g. size, volume and/or location) information relating to each lesion. For example, a PI-RADS score given to a lesion identified in an image may be combined with information describing an estimated volume of the tumor and/or with information describing the location of the lesion in the tumor (e.g. located in the anterior part of the tumor or in the posterior part of the tumor). Thus, "spatial" information may be considered to include morphological information, such as volume, dimensions, and structure. In one example, the volume may be determined by a radiologist delineating the lesion in an MR image such that the volume of the lesion can be calculated. PI-RADS scores may be assigned to lesions identified in images on a region-by-region basis. For example, an MR image of the prostate may be divided into a number of regions or zones, and a PI-RADS score may be given for each zone. In some examples, regions of the prostate may be identified as an anterior fibromuscular stroma, a transition or transitional zone (TZ), a central zone (CZ) and a peripheral zone (PZ). The PI-RADS scores may be combined with the zone in which the PI-RADS score is assigned in order to perform the prognostic evaluation. The PI-RADS scores may be combined with details of a region (e.g. the anterior or posterior region) in which the PI-RADS score is assigned in order to perform the prognostic evaluation. The imaging data 106 may, in some embodiments, include apparent diffusion coefficient (ADC) values relating to a lesion associated with prostate cancer.

The pathological information 108 may include data obtained or acquired from a biopsy performed on the tumor in the subject's prostate. A biopsy of the tumor in the prostate is assessed or analyzed by one or more medical professionals, and given a score according to the Gleason grading system. A Gleason score is given to a cancerous tumor in the prostate based on its microscopic appearance, and is indicative of the aggressiveness of the cancer. A Gleason score (also referred to as the Gleason sum) ranges from 2 to 10, and is calculated by adding together primary and secondary Gleason grades, each ranging from 1 to 5. The first half of the score, referred to as primary Gleason grade, is based on the dominant cellular de-differentiation pattern (i.e. the cellular de-differentiation pattern constituting over than 50% of the total pattern) in the tumor, and the second half of the score, referred to as the secondary Gleason grade, is based on the next-most frequent cellular de-differentiation pattern (i.e. a non-dominant cellular de-differentiation pattern) in the tumor. The primary Gleason grade and the secondary Gleason grade may be used in order to perform the prognostic evaluation.

The data 102, 104, 106, 108 may, example, be stored in a storage unit, such as a memory, accessible by the prognostic evaluation unit 100. In some examples, such a memory may include a database containing data for a plurality of subjects suffering from prostate cancer. The prognostic evaluation unit 100 may retrieve the data for a particular subject from the memory or database, and process the data in order to perform a prognostic evaluation.

The prognostic evaluation unit 100 is configured to evaluate the data 102, 104, 106, 108 and determine a prognostic score relating to the cancer of the particular subject to whom the data relates. As shown in Fig. 1, the prognostic evaluation unit 100 may, in some embodiments, generate two scores as its output. A first score 110 may comprise a score indicative of the likelihood that cancer will be clinically significant after a radical prostatectomy (i.e. removal of the whole prostate) has been performed. In some embodiments, the cancer would be considered to be clinically significant if, after a radical prostatectomy, any remaining cancer (i.e. a tumor) would be scored greater than or equal to 7 according to the Gleason grading system. A second score 112 may comprise a score indicative of the likelihood that the cancer is non-localized after a radical prostatectomy has been performed. In some embodiments, the cancer would be considered to be non-localized if, after a radical prostatectomy, any remaining cancer would be assigned a pathologic stage of greater than pT2.

In some embodiments, the prognostic evaluation unit 100 may comprise a prediction model (e.g. a classifier, regression model, and the like) arranged to process the input data 102, 104, 106, 108 in order to generate a prognostic score relating to the cancer. Such a classifier may, in some embodiments, be obtained by training a machine learning model or algorithm using training data. For example, the classifier may be based on a random forest model or a regression model, such as a linear regression model or a logistic regression model. Embodiments in which a random forest model is trained to function as the classifier are discussed in greater detail below.

The prognostic score (which may include the first score 110 and/or the second score 112) may provide a medical professional with an accurate insight into the prognosis of the subject in view of the prostate cancer, based on the data provided. The prognostic score may be used by a medical professional to determine an appropriate course of action for the subject. For example, if it is determined from the prognostic score that the subject has primary localized prostate cancer, then a medical professional may recommend that the subject undergoes active surveillance (i.e. regular monitoring) rather than active treatment, which may include surgery or radiation therapy, for example. Determining a course of action without using the prognostic evaluation unit 100 may result in active treatment being recommended for a subject for whom active surveillance would be suitable. Thus, the prognostic evaluation unit 100 may result in fewer subjects being unnecessarily treated using active treatment. This, in turn, may reduce the amount of resources (both medical professionals and medical equipment in a medical facility) required, and may lead to cost savings for the medical facility treating the subject.

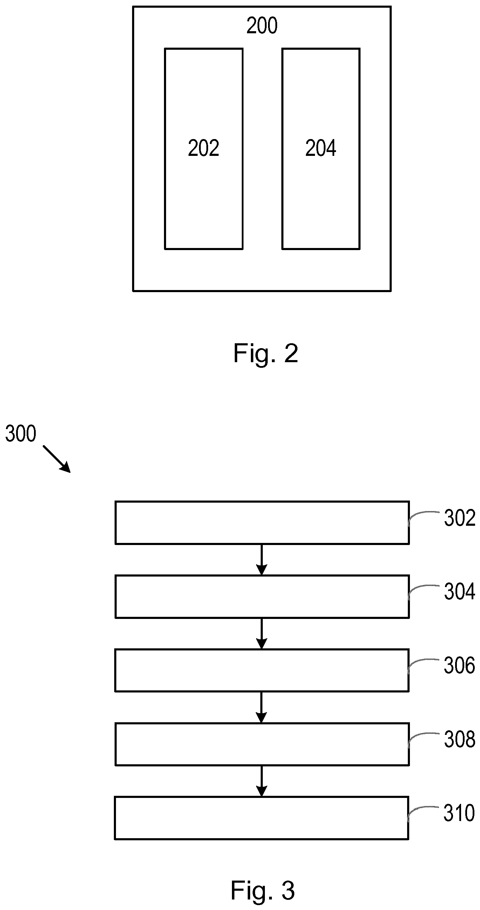

According to an aspect, embodiments disclosed herein relate to an apparatus for performing a prognostic evaluation of a subject potentially having prostate cancer. In some embodiments, the subject may suffer from prostate cancer. Fig. 2 is a simplified schematic of such an apparatus 200. The apparatus 200 may be embodied as a computing device in some examples. The apparatus 200 comprises a memory 202 and a processor 204. The memory 202 comprises instruction data representing a set of instructions. The processor is configured to communicate with the memory 202 and to execute the set of instructions. The set of instructions, when executed by the processor 204, cause the processor to perform the methods disclosed herein.

In some embodiments, the set of instructions, when executed by the processor 204, cause the processor to obtain a subject profile associated with the subject. The subject profile may, for example, comprise the subject profile 102 discussed above. As noted above, the subject profile may include characteristics of the subject, such as the subject's age and/or details of any occurrences of cancer in members of the subject's family (i.e. a family history of cancer).

In some embodiments, the set of instructions, when executed by the processor 204, cause the processor to obtain clinical data associated with the subject. The clinical data may, for example, comprise the clinical data 104 discussed above. As noted above, the clinical data may, in some embodiments, comprise one or more of: prostate-specific antigen (PSA) density data (i.e. data indicating a PSA density detected in the prostate); and clinical tumor stage information (i.e. an indication of the clinical tumor stage, or the cT class).

In some embodiments, the set of instructions, when executed by the processor 204, cause the processor to obtain imaging data acquired in respect of the subject's prostate. The imaging data may, for example, comprise the imaging data 106 discussed above. As noted above, the imaging data may, in some embodiments, comprise one or more of: a Prostate Imaging Reporting and Data System (PI-RADS) score relating to the prostate cancer; and spatial information relating to a lesion associated with the prostate cancer. The PI-RADS score and the spatial information (which may include location information and morphologic information, such as volume and structure/shape) may be derived from multi-parametric magnetic resonance imaging (MRI) information. Thus, the imaging data may include PI-RADS scores given to lesions visible in the imaging data. In some embodiments, the imaging data may comprise a PI-RADS score relating to each of one or more lesions associated with the prostate cancer, each PI-RADS score determined using one or more of T2-weighted (T2-w) image data, diffusion-weighted image (DWI) data and dynamic contrast-enhanced (DCE) image data. The spatial information may include location information (e.g. anterior or posterior) relating to the lesions visible in the imaging data for which PI-RADS scores have been given. The spatial information may, in some embodiments, include an indication of a size of the one or more lesions in a peripheral zone of the prostate, in a central zone of the prostate, in a transition zone of the prostate and/or in an anterior fibromuscular stroma of the prostate. The spatial information (e.g. the morphological information) may also include data indicative of the size of the tumor in the prostate.

In some embodiments, the set of instructions, when executed by the processor 204, cause the processor to obtain pathological information relating to a biopsy acquired in respect of the subject's prostate. The pathological information may, for example, comprise the pathological information 108 discussed above. As noted above, the pathological information may include data determined from a biopsy of the subject's prostate. For example, the pathological information may include at least one of a primary Gleason score and a secondary Gleason score given based on an examination of the biopsy.

The processor 204 may obtain the various items of data (i.e. the subject profile, the clinical data, the imaging data and/or pathological information) by retrieving the data from one or more databases or by a user manually inputting the data, for example via a user interface associated with the apparatus 200.

In some embodiments, the set of instructions, when executed by the processor 204, cause the processor to determine, based on at least the subject profile, the clinical data, the imaging data and the pathological information, a prognostic score relating to the cancer. As noted briefly above, and discussed in greater detail below, the prognostic score may be determined using a prediction model, classifier, or algorithm. For example, a model may be trained using machine learning techniques, and used to determine the prognostic score based on the input data. In some embodiments, the prognostic score may be determined using a trained random forest classify, a linear regression analysis model or a logistic regression analysis model. In other examples, another type of analysis may be performed in order to determine the prognostic score.

The determined prognostic score may, in some embodiments, comprise a score relating to the likelihood that the cancer is clinically significant. As noted above, the cancer may be considered to be clinically significant if, following a radical prostatectomy, any remaining cancer would be given the Gleason sum score of at least 7. In some embodiments, the determined prognostic score may comprise a score relating to the likelihood that the cancer is non-localized. A cancer may be non-localized if it has spread beyond the prostate to another part of the subject's body, for example by metastases. The cancer may be considered to be non-localized if, following a radical prostatectomy, a pathologic stage relating to any remaining cancer would be greater than pT2. In some embodiments, the prognostic score determined by the processor 204 may include multiple scores (e.g. a score relating to the likelihood that the cancer is clinically significant and a score relating to the likelihood that the cancer is non-localized). The prognostic score determined by the processor 204 may, in some embodiments, include a combined score taking into account the likelihood that the cancer is clinically significant and the likelihood that the cancer is non-localized. In some examples, the prognostic score may be a numerical score (or multiple numerical scores) on a scale of 0 to 10, or to 100, for example, or a decimal score on a scale of 0 to 1, or 0 to 100, for example.

According to a further aspect, embodiments relate to a method of performing a prognostic evaluation of a subject having prostate cancer. Fig. 3 is a flowchart of an example of such a method 300. The method 300 comprises, at step 302, obtaining a subject profile associated with the subject. At step 304, the method 300 comprises obtaining clinical data associated with the subject. The method 300 comprises, at step 306, obtaining imaging data acquired in respect of the prostate. At step 308, the method 300 comprises obtaining pathological information relating to a biopsy acquired in respect of the subject's prostate. The method 300 comprises, at step 310, determining, based on at least the subject profile, the clinical data, the imaging data and the pathological information, a prognostic score relating to the cancer. The method 300 may, in some examples, be performed by the apparatus 200 discussed above.

In some embodiments, the step 310 of determining a prognostic score may comprise providing the subject profile, the clinical data, the imaging data and the pathological information as inputs to a prediction model, such as a trained classifier. The determining 310 may further comprise obtaining as an output at least one of: a score indicating the likelihood that the cancer is clinically significant; and a score indicating the likelihood that the cancer is non-localized.

The method 300 may, in some embodiments, further comprise a step of delivering the prognostic score for presentation or display to a user (e.g. a medical professional). The score may, for example, be presented on a display associated with the apparatus 200.

As noted previously, determining the prognostic score may be performed using a prediction model which may, in some embodiments, be a trained classifier, such as a random forest classifier, a linear regression analysis model or a logistic regression analysis model. The according to some embodiments, the prediction model may be trained using a training mechanism which improves (and possibly optimizes) the model such that the determination of the prognostic score is accurate and meaningful.

In some embodiments, the prediction model may comprise a random forest classifier. A random forest classifier (also referred to as a random decision forest) will be familiar to skilled in the field of machine learning. Briefly, the random forest classifier is an ensemble algorithm combining a multitude of trained decision trees that vary slightly from one another by random sampling. The random forest classifier is able to accurately predict an outcome (e.g. provide a prognostic score) based on a plurality of input features (e.g. the input data 102, 104, 106, 108). The output is the mode of the classes determined during the classification. During training of a random forest classifier, the classifier provides an internal ranking of a feature importance. In other words, when training a random forest classifier, it is possible to identify those features which have the greatest effect on the output and those features which have the least effect on the output. Features which have little effect on the output may still have a negative effect on the overall discriminating performance if they are taken into account by the classifier. Therefore, according to some embodiments, the lowest ranking features (i.e. those features having the least effect on the output of the classifier) are removed such that the classifier no longer takes those features into account. The removal of the lowest ranking feature may be referred to as "worst-first-out".

The prediction model may, in some embodiments, be trained using a set of training data which includes subject profile information, clinical data, imaging data and pathological information which has been verified by one or more medical professionals (e.g. radiologists). For example, in some embodiments, the prediction model may be trained using some or all of the following features:
1. PI-RADS score of the most dominant lesion in a T2-w image;
2. PI-RADS score of the most dominant lesion in a DWI image;
3. PI-RADS score of the most dominant lesion in a DCE image;
4. An indication of the size of the tumor in the prostate (e.g. an indication of the size of all of the lesions for which a PI-RADS score has been determined);
5. An indication of the size of all lesions identified by a radiologist in an anterior region of the prostate;
6. An indication of the size of all lesions identified by radiologist in a posterior region of the prostate;
7. An indication of the size of all lesions identified by radiologist in the anterior fibromuscular stroma of the prostrate;
8. An indication of the size of all lesions identified by radiologist in the transitional zone (TZ) of the prostrate;
9. An indication of the size of all lesions identified by radiologist in the the central zone (CZ) of the prostate;
10. An indication of the size of all lesions identified by radiologist in the peripheral zone (PZ) of the prostate;
11. Clinical tumor stage (cT);
12. Primary Gleason biopsy score;
13. Secondary Gleason biopsy score;
14. PSA density (calculated by the ratio of PSA level and prostate volume); and
15. Age of the subject at the time of diagnosis.

Thus, for each case included in the training data, one or more radiologists determine some or all of the above features (1 to 15), and these are provided to the prediction model to enable the model to learn based on these features.

As will be familiar to those skilled in the art, PI-RADS scores may be given based on various versions of the PI-RADS scoring system. According to embodiments disclosed herein, the PI-RADS version 1 (v1) scoring system or the PI-RADS version 2 (v2) scoring system may be used. Scores given using the PI-RADS v1 system, may be converted into equivalent scores in the PI-RADS v2 system as described above.

When training the prediction model using a large set of training data, there is a chance that the majority class (i.e. the class most commonly output by the model which, in context of prostate cancer patients, is likely to be the class representing benign and/or non-aggressive cancer) is dominant. Using a training data set having a large majority class (and a relatively small minority class) will cause the model to bias towards the majority class, leading to an over-optimistic discriminative performance. Thus, to reduce this effect, action may, in some embodiments, be taken to statistically balance the dataset. In some embodiments, the majority class and minority class of the training data set may be balanced using an adapted form of bagging. Thus, a statistically balanced dataset is created by down-sampling (or under-sampling) the majority class in the training data set. Thereafter, a bootstrapping technique (also referred to as bootstrap sampling) is applied to train a multitude of random forests on a multitude of balanced datasets such that all cases in the majority class are included for training. The multitude of random forests is combined in a single random forests classifier. Thus, the majority class is randomly down-sampled to make it equal in size to the minority class. This balanced data set is used to train the first random forest. A second iteration is then performed, whereby the majority class is randomly down-sampled, creating a new balanced data set, which is used to train the second random forest. This down-sampling/training process is repeated N times until at least all the cases in the majority class have been iterated (i.e. bootstrapped).

As noted briefly above, the random forest classifier automatically performs feature ranking during training. Embodiments disclosed herein make use of this feature ranking so that the effect of those features that have a positive impact on the discriminating performance of the classifier can be increased and the effect of those features that have a negative impact in the discriminating performance of the classifier can be decreased. According to some embodiments, the impact of features may be assessed by analyzing an area under the receiver operating curve (ROC) for each feature. After a first round of training, the feature having the smallest area under the curve may be considered to have the smallest effect on the output of the classifier and, therefore, that feature may be removed and not take into account by the classifier in future rounds of training. The lowest-ranked features may be removed one-by-one in an iterative process until the removal of an additional feature has no positive effect on the discriminative performance of the classifier (i.e. until the removal of the lowest-ranked feature does not lead to an improvement in the performance of the classifier). The remaining subset of features may be considered to constitute an optimal feature subset to be used by the classifier. When the classifier is to be used, it may process the input data based on the optimal feature subset in order to provide an optimum output. Thus, the effect of removing the lowest-ranked (i.e. the worst-performing) features is that the accuracy of the classifier is improved.

Referring again to Fig. 2, the memory 202 of the apparatus 200 may comprise a set of instructions which, when executed by the processor 204, cause the processor to determine the prognostic score by using a trained random forest classifier. The random forest classifier may be arranged to classify input data based on a subset of a plurality of features. The subset of features may include those feature of the plurality of features which have the greatest impact in the classification. In other words, the subset of features may include those features which have not been removed from the plurality of features in the process described above.

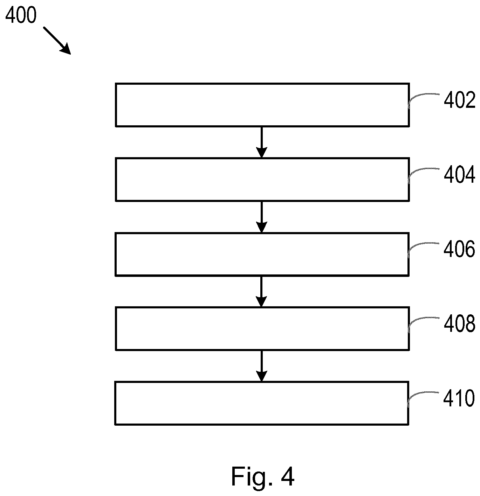

Referring now to Fig. 4, an aspect disclosed herein relates to a computer-implemented method of training a classifier. Fig. 4 is a flowchart of an example of such a method 400. The method 400 is a method of training a classifier to determine a prognostic score relating to prostate cancer in a subject based on a subject profile associated with the subject, clinical data associated with the subject, imaging data acquired in respect of the prostate and pathological information relating to a biopsy acquired in respect of the subject's prostate. The method 400 comprises, at step 402, training the classifier using a set of training data. At step 404, the method 400 comprises identifying a plurality of features which affect the output of the classifier. The method 400 comprises, at step 406, ranking the plurality of features according to their impact on the output of the classifier. At step 408, the method 400 comprises disregarding the lowest-ranked feature of the plurality of features to obtain a subset of higher-impact features. Step 408 may comprise the removal of the lowest-ranked feature (i.e. the worst performing feature) as described above. The method 400 comprises, at step 410, re-training the classifier on the basis of the subset of features. Here, the term "re-training" refers to performing a further round of training on the basis of the reduced subset of features. Thus, the method 400 may be considered to repeat steps 402, 404, 406 and 408 in a loop until an stop criterion is met (e.g. until the highest area under the receiver operating curve (ROC) is obtained.

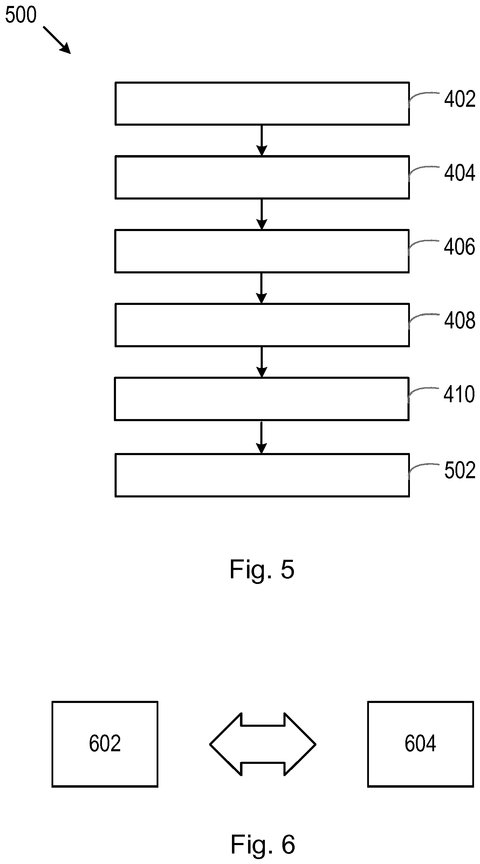

Fig. 5 is a flowchart of a further example of a method 500 of training a classifier. The method 500, which may be computer-implemented, may include steps of the method 400, such as steps 402 to 410. The method 500 may further comprise, at step 502, down-sampling a majority class of the training data used to train the classifier such that the majority class is statistically balanced with a minority class of the training data. Training the classifier may comprise bootstrap sampling data in the minority class of the training data. Step 502 may, for example, be performed before step 402 of the method 400.

According to a further aspect, a computer program product is disclosed. Fig. 6 is a simplified schematic of an example of a machine-readable medium 602 in communication with a processor 604. The computer program product comprises a non-transitory computer-readable medium (e.g. the machine-readable medium 602) having computer-readable code embodied therein. The computer readable code is configured such that, on execution by a suitable computer or processor (e.g. the processor 604), the computer or processor is caused to perform steps of any of the methods (300, 400, 500) disclosed herein. In some examples, the processor 604 may comprise or be similar to, the processor 204 discussed above.

The processor 204, 604 can comprise one or more processors, processing units, multi-core processors or modules that are configured or programmed to control the apparatus 200 in the manner described herein. In particular implementations, the processor 204, 604 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (200) for performing a prognostic evaluation of a subject potentially having prostate cancer, the apparatus comprising:
a memory (202) comprising instruction data representing a set of instructions; and
a processor (204) configured to communicate with the memory and to execute the set of instructions, wherein the set of instructions, when executed by the processor, cause the processor to:
obtain a subject profile associated with the subject;
obtain clinical data associated with the subject;
obtain imaging data acquired in respect of the subject's prostate;
obtain pathological information relating to a biopsy acquired in respect of the subject's prostate; and
determine, based on at least the subject profile, the clinical data, the imaging data and the pathological information, a prognostic score relating to the cancer.

2. An apparatus (200) according to claim 1, wherein the determined prognostic score comprises a score relating to the likelihood that the cancer is clinically significant.

3. An apparatus (200) according to any of the preceding claims, wherein the determined prognostic score comprises a score relating to the likelihood that the cancer is non-localized.

4. An apparatus (200) according to claim 3, wherein the cancer is considered to be non-localized if, following a radical prostatectomy, a pathologic stage relating to any remaining cancer would be greater than pT2.

5. An apparatus (200) according to any of the preceding claims, wherein the subject profile comprises one or more of: an age of the subject; a family history of cancer; demographic data for the subject; an ethnic background of the subject; information relating to comorbidities of the subject; and a treatment history of the subject.

6. An apparatus (200) according to any of the preceding claims, wherein the clinical data comprises one or more of: prostate-specific antigen density data; clinical tumor stage information; data relating to an outcome of a digital rectal exam; and data relating to an outcome of a transrectal ultrasound.

7. An apparatus (200) according to any of the preceding claims, wherein the imaging data comprises one or more of: a Prostate Imaging Reporting and Data System, PI-RADS, score relating to the prostate cancer; spatial information relating to a lesion associated with the prostate cancer; and apparent diffusion coefficient values relating to a lesion associated with prostate cancer;
wherein the PI-RADS score and the spatial information are derived from multi-parametric magnetic resonance imaging information.

8. An apparatus (200) according to claim 7, wherein the imaging data comprises a PI-RADS score relating to each of one or more lesions associated with the prostate cancer, each PI-RADS score determined using one or more of T2-weighted image data, diffusion-weighted image data and dynamic contrast-enhanced image data; and
wherein the spatial information comprises an indication of a size of the one or more lesions in total, in an anterior region of the subject's prostate, in a posterior region of the subject's prostate; in a peripheral zone of the prostate, in a central zone of the prostate, in a transition zone of the prostate and/or in an anterior fibromuscular stroma of the prostate.

9. An apparatus (200) according to any of the preceding claims, wherein the set of instructions, when executed by the processor, cause the processor to determine the prognostic score by using a prediction model.

10. An apparatus (200) according to any of the preceding claims, wherein the set of instructions, when executed by the processor, cause the processor to determine the prognostic score by using a trained random forest classifier; wherein the random forest classifier is arranged to classify input data based on a subset of a plurality of features; and
wherein the subset of features includes those feature of the plurality of features which have the greatest impact in the classification.

11. A computer-implemented method (300) of performing a prognostic evaluation of a subject potentially having prostate cancer, the method comprising:
obtaining (302) a subject profile associated with the subject;
obtaining (304) clinical data associated with the subject;
obtaining (306) imaging data acquired in respect of the prostate;
obtaining (308) pathological information relating to a biopsy acquired in respect of the subject's prostate; and
determining (310), based on at least the subject profile, the clinical data, the imaging data and the pathological information, a prognostic score relating to the cancer.

12. A computer-implemented method according to claim 11, wherein determining (310) a prognostic score comprises:
providing the subject profile, the clinical data, the imaging data and the pathological information as inputs to a prediction model; and
obtaining as an output at least one of: a score indicating the likelihood that the cancer is clinically significant; and a score indicating the likelihood that the cancer is non-localized.

13. A computer-implemented method (400) of training a classifier to determine a prognostic score relating to prostate cancer in a subject based on a subject profile associated with the subject, clinical data associated with the subject, imaging data acquired in respect of the prostate and pathological information relating to a biopsy acquired in respect of the subject's prostate, the method comprising:
training (402) the classifier using a set of training data;
identifying (404) a plurality of features which affect the output of the classifier;
ranking (406) the plurality of features according to their impact on the output of the classifier;
disregarding (408) the lowest-ranked feature of the plurality of features to obtain a subset of higher-impact features; and
re-training (410) the classifier on the basis of the subset of features.

14. A method (400, 500) according to claim 13, further comprising:
down-sampling (502) a majority class of the training data used to train the classifier such that the majority class is statistically balanced with a minority class of the training data;
wherein training the classifier comprises bootstrap sampling data in the minority class of the training data.

15. A computer program product comprising a non-transitory computer-readable medium, the computer-readable medium (602) having computer-readable code embodied therein, the computer-readable code being configured such that, on execution by a suitable computer or processor (604), the computer or processor is caused to perform the method of any of claims 11 to 14.
